# EUROPEAN PATENT SPECIFICATION

(11) **EP 4 232 557 B1**
(45) Date of publication and mention of the grant of the patent: **11.03.2026**
(21) Application number: 21791417.5
(22) Date of filing: 20.10.2021
(51) Int. Cl.: C12N 5/00

(54) **POLOXAMERS FOR CELL CULTURE**
POLOXAMERE FÜR ZELLKULTUR
POLOXAMÈRES POUR CULTURE CELLULAIRE

(30) Priority: 21.10.2020 EP 20203086
(43) Date of publication of application: 30.08.2023
(73) Proprietor: Merck Patent GmbH, 64293 Darmstadt (DE)
(72) Inventor: ANTONELLO, Alice, 64293 Darmstadt (DE); FEIGENSPAN, Melanie, 64293 Darmstadt (DE); RAPP, Almut, 64293 Darmstadt (DE); SCHULTHEISS, Christian, 64293 Darmstadt (DE); SIECK, Jochen Bastian, 64293 Darmstadt (DE)
(74) Representative: Merck Patent Association
(86) International application number: PCT/EP2021/079085
(87) International publication number: WO 2022/084386

(56) References cited:
- WO-A1-2015/003773
- WO-A2-03/084479
- MICHAEL LINN ET AL: "Soluplus as an effective absorption enhancer of poorly soluble drugs in vitro and in vivo", EUROPEAN JOURNAL OF PHARMACEUTICAL SCIENCES, ELSEVIER, AMSTERDAM, NL, vol. 45, no. 3, 14 February 2012 (2012-02-14), pages 336 - 343, XP028440388, ISSN: 0928-0987, [retrieved on 20111208], DOI: 10.1016/J.EJPS.2011.11.025
- HAOFAN PENG ET AL: "Mechanism investigation for poloxamer 188 raw material variation in cell culture", BIOTECHNOLOGY PROGRESS, vol. 32, no. 3, 1 May 2016 (2016-05-01), pages 767 - 775, XP055545565, ISSN: 8756-7938, DOI: 10.1002/btpr.2268
- SAKAI KENTARO ET AL: "Use of nonionic surfactants for effective supply of phosphatidic acid in serum-free culture of Chinese hamster ovary cells", JOURNAL OF BIOSCIENCE AND BIOENGINEERING, ELSEVIER, AMSTERDAM, NL, vol. 92, no. 3, 1 January 2001 (2001-01-01), pages 256 - 261, XP002255475, ISSN: 1389-1723, DOI: 10.1263/JBB.92.256
- CHANG DAVID ET AL: "Investigation of interfacial properties of pure and mixed poloxamers for surfactant-mediated shear protection of mammalian cells", COLLOIDS AND SURFACES B: BIOINTERFACES, ELSEVIER AMSTERDAM, NL, vol. 156, 16 May 2017 (2017-05-16), pages 358 - 365, XP085082047, ISSN: 0927-7765, DOI: 10.1016/J.COLSURFB.2017.05.040
- THIMMASANDRA NARAYANAPPA ATHMARAM: "A rapid cell-based assay for determining poloxamer quality in CHO suspension cell culture", BIOTECHNIQUES, vol. 67, no. 3, 1 September 2019 (2019-09-01), pages 98 - 109, XP055878516, Retrieved from the Internet <URL:https://www.future-science.com/doi/pdf/10.2144/btn-2019-0070>

## Description

The present invention relates to the use of poloxamers as cell culture media additives. The poloxamers are suitable for foam reduction as well as for shear protection.

### Background of the invention

Poloxamers, especially Poloxamer 188, are used in many industrial applications, cosmetics, and pharmaceuticals. They are also used in cell culture media processes. The addition of poloxamers, especially poloxamer 188, to cell culture media improves cell viability significantly. High cell viability is crucial for optimal protein production. Why poloxamers improve cell viability is not fully understood. It is believed that poloxamers reduce shear stress and in this way protect the cells from damage. Poloxamer, being a nonionic surfactant, is likely to concentrate at the gas bubble/medium interface and could prevent cell attachment to gas bubbles and in this way prevent cells from damage when gas bubbles burst. It may also reduce shock when bubbles burst. Some publications claim that poloxamers improve the oxygen transfer rate from the gas into the liquid phase, but other publications contradict these findings. There are also indications that poloxamers may "repair" small defects in cell membranes.

Unfortunately, Poloxamer 188 contributes significantly to stabilize the foam produced in sparged bioreactors. Therefore, a non- or low-foaming alternative to Poloxamer 188 would be desirable.

Poloxamers with a high propylene oxide/ethylene oxide ratio and thus high hydrophobicity, are known to be good defoamers as described by Schmolka I. R. (Schmolka, Journal of the American Oil Chemists' Society (1977), 54(3), 110-16). Poloxamer 101, 181, 182 or 184 are examples of such defoaming poloxamers. But poloxamer 181 was described by Murhammer (D. W. Murhammer, C. F. Gochee, Biotechnol. Prog. 1990, 6, 142-146) of being toxic for insect cells. Poloxamer 105 was described by Murhammer, see above, of generating less foam than poloxamer 188 and not inhibiting cell growth of insect cells. Murhammer postulates a correlation between reduced foam formation and reduced polymer length. But problems with toxicity also need to be taken into account. Plenty of poloxamers with an average molecular weight around 2000 like Poloxamer 105 are toxic for cells.

WO03/084479 discloses a method for stabilizing cells like CHO cells during cultivation using different shear-protective compounds.

Chang, David et al. Colloids and Surfaces B Biointerfaces, Vol. 156, 16 May 2017, pages 358-365 discuss the effect of shear stress protection and foam stability of poloxamer 188 and poloxamer 407.

Haofang, Peng et al., Biotechnology Progres, Vol. 32, No. 3, 1 May 2016, pages 767-775 disclose the use of poloxamer 188 in cell culture to protect cells from hydrodynamic shear forces.

It has now been found that certain poloxamers fulfill all requirements concerning toxicity, foam formation, and shear stress protection - independent from their molecular weight. The requirements for an ideal suitability in cell culture are a certain PPO (polypropylene oxide) block length combined with a defined percentage of the PEO (polyethylene oxide) part. It has been found that the molecular weight of the PPO block correlates with the maximum foam height. For poloxamers with an ethylene oxide percentage of >45%, the higher the molecular weight of the PPO block, the higher the maximum foam height is. It has further been found that poloxamers with an ethylene oxide percentage of >45% (w/w) and a polypropylene oxide block <700 g/mol are well suited as a low foaming alternative to poloxamer 188 in cell culture since they perform well also as shear-stress protectors and are typically non-toxic.

The present invention is thus directed to a cell culture medium comprising a poloxamer with a polypropylene oxide block <700 g/mol and an ethylene oxide percentage of >45%, whereby the cell culture medium comprises the poloxamer in an amount between 0.1 to 10 g/L calculated for the liquid medium.

In a preferred embodiment, the molecular weight of the PPO block is between 300 and 700 g/mol.

In a preferred embodiment, the PEO percentage is between 47 and 90%.

In a very preferred embodiment, the polyethylene oxide percentage is between 75-90% (w/w) and the molecular weight of the PPO block is between 300 and 700 g/mol.

Poloxamers with the described attributes may be liquid or solid at room temperature, in a very preferred embodiment the poloxamers are solid at room temperature so that they can for example be milled and treated equivalent to other dry powder media ingredients.

In a very preferred embodiment, the amount of poloxamer is between 0.5 to 2 g/L calculated for the liquid medium.

In one embodiment the cell culture medium is a chemically defined medium.

In one embodiment the cell culture medium is a dry powder or a dry compacted medium.

In another embodiment, the cell culture medium comprises at least one or more saccharide components, one or more amino acids, one or more vitamins or vitamin precursors, one or more salts, one or more buffer components, one or more co-factors and one or more nucleic acid components.

The present invention is also directed to a process for culturing cells whereby the cells are cultured in a liquid medium comprising a poloxamer with a polypropylene oxide block <700 g/mol and an ethylene oxide percentage of >45%. Preferably the process includes agitation and/or sparging.

In a preferred embodiment, the molecular weight of the PPO block is between 300 and 700 g/mol.

In a very preferred embodiment, the ethylene oxide percentage is between 75-90% (w/w) and the molecular weight of the PPO block is between 300 and 700 g/mol.

In a preferred embodiment, the amount of antifoaming agent in the liquid medium is reduced compared to a cell culture medium that is otherwise identical but comprises poloxamer 188 instead of a poloxamer with a polypropylene oxide block <700 g/mol and an ethylene oxide percentage of >45%.

The present invention is also directed to a method for reducing foam formation in agitated and/or sparged cell cultures whereby the cells are cultured in a liquid medium comprising a poloxamer with a polypropylene oxide block <700 g/mol and an ethylene oxide percentage of >45% and the foam formation is reduced compared to cells cultured under the same conditions in a cell culture medium that comprises poloxamer 188 instead of a poloxamer with a polypropylene oxide block <700 g/mol and an ethylene oxide percentage of >45%.

### Figures

Figures 1 and 2 show the results of toxicity assays. Further details can be found in Example 1.
Figure 3 shows the correlation between cell viability and %EO in poloxamer. Further details can be found in Example 1.
Figures 4 and 5 show the results of foam evolution during continuous sparging. Further details can be found in Example 2.
Figure 6 shows the correlation between average maximum foam height as well as average plateau height and molecular weight of the polypropylene oxide block in poloxamers. Further details can be found in Example 2.
Figure 7 shows foam evolution for different concentrations of Poloxamer 65. Further details can be found in Example 2.
Figure 8 shows the results of a shear stress assay. Further details can be found in Example 3.
Figure 9 shows the Molecular weight distribution determined with size exclusion chromatography (see Method 1) for poloxamer 79a. Mp indicates the peak molecular weight of the specific poloxamer.
Figure 10 shows the results of the fed-batch cell culture. Further details can be found in Example 4.

### Definitions

Before describing the present invention in detail, it is to be understood that this invention is not limited to specific compositions or process steps, as such may vary. It must be noted that, as used in this specification and the appended claims, the singular form "a", "an" and "the" include plural referents unless the context clearly dictates otherwise. Thus, for example, reference to "a poloxamer" includes a plurality of poloxamers and the like. Unless defined otherwise, all technical and scientific terms used herein have the same meaning as commonly understood by one of ordinary skill in the art to which this invention is related. The following terms are defined for purposes of the invention as described herein.

The term "bioreactor," as used herein, refers to any manufactured or engineered device or system that supports a biologically active environment. In some instances, a bioreactor is a vessel or tank in which a cell culture process is carried out which involves organisms or biochemically active substances derived from such organisms. Such a process may be either aerobic or anaerobic. Commonly used bioreactors are typically cylindrical, ranging in size from liters to cubic meters, and are often made of stainless steel. In some embodiments described herein, a bioreactor might contain a disposable constituent made of a material other than steel and is disposable. In some embodiments that is a disposable bag where in the biologically active environment is maintained. It is contemplated that the total volume of a bioreactor may be any volume ranging from 100 mL to up to 10,000 Liters or more, depending on a particular process.

An agitated cell culture is a cell culture that is performed in a bioreactor by permanently or one or several times during the cell culture agitating the cell culture medium with the cells in the bioreactor.

Agitation can for example be done by stirring, rocking, or shaking. In stirred tank bioreactors, one or several impellers may be deployed, depending on the geometry of the vessels. Stirrer types will be selected according to the main mixing task and requirements of the process.

A sparged cell culture is a cell culture in which a gas is introduced into the bioreactor. This is typically done with a sparger, also called bubbler or aerator.

Typically, in a sparged cell culture, a mixture of air, oxygen, carbon dioxide, and nitrogen is introduced into the bioreactor with a sparger. Typical spargers for cell culture processes include drilled-hole ring spargers, sintered micro-spargers, open pipe spargers, and hybrid forms. Surface aeration also contributes to oxygen transfer, but the impact of surface aeration decreases during scale-up. Both stirrer and sparger types in mammalian cell culture processes will often be selected according to the cells' shear sensitivity (Nienow, A.W., 2006. Reactor engineering in large scale animal cell culture, Cytotechnology, 50(1-3), p.9).

A cell culture medium according to the present invention is any mixture of components which maintains and/or supports the in vitro growth of cells and/or supports a particular physiological state, typically by providing at least one nutrient to the cells. It might be a complex medium or a chemically defined medium. The cell culture medium can comprise all components necessary to maintain and/or support the in vitro growth of cells or only some components so that further components are added separately. Examples of cell culture media according to the present invention are full media which comprise all components necessary to maintain and/or support the in vitro growth of cells as well as media supplements or feeds. In a preferred embodiment, the cell culture medium is a full medium, a perfusion medium or a feed medium. A full medium also called basal medium typically has a pH between 6.7 and 7.8. A feed medium preferably has a pH below 8.5.

Typically, the cell culture media according to the invention are used to maintain and/or support the growth of cells in a bioreactor.

A feed or feed medium is a cell culture medium that is not the basal medium that supports initial growth and production in cell culture but the medium which is added at a later stage to prevent depletion of nutrients and sustains the production phase. A feed medium can have higher concentrations of some components compared to a basal culture medium. For example, some components, such as, for example, nutrients including amino acids or carbohydrates, may be present in the feed medium at about 5X, 6X, 7X, 8X, 9X, 10X, 12X, 14X, 16X, 20X, 30X, 50X, 100x, 200X, 400X, 600X, 800X, or even about 1000X of the concentrations in a basal medium.

A mammalian cell culture medium is a mixture of components which maintain and/or support the in vitro growth of mammalian cells. Examples of mammalian cells are human or animal cells, preferably CHO cells, COS cells, I VERO cells, BHK cells, AK-1 cells, SP2/0 cells, L5.1 cells, hybridoma cells, or human cells.

Chemically defined cell culture media are cell culture media that do not comprise any chemically undefined substances. This means that the chemical composition of all the chemicals used in the media is known. The chemically defined media do not comprise any yeast, animal, or plant tissues; they do not comprise feeder cells, serum, hydrolysates, extracts or digests or other poorly defined components. Chemically undefined or poorly defined chemical components are those whose chemical composition and structure are not known, are present in varying composition or could only be defined with enormous experimental effort - comparable to the evaluation of the chemical composition and structure of a protein like insulin, albumin or casein.

A powdered cell culture medium or a dry powder medium is a cell culture medium typically resulting from a milling process or a lyophilization process. That means the powdered cell culture medium is a granular, particulate medium - not a liquid medium. The term "dry powder" may be used interchangeably with the term "powder;" however, "dry powder" as used herein simply refers to the gross appearance of the granulated material and is not intended to mean that the material is completely free of complexed or agglomerated solvent unless otherwise indicated.

A dry granulated medium is a dry medium resulting from a wet or dry granulation process, e.g. by spray drying, wet granulation, or dry compaction, typically have particle sizes above 0.5 mm, e.g. between 0.5 and 5 mm. Dry compaction is typically done in a roll press. US 6,383,810 B2 discloses a method of producing a wet granulated eukaryotic cell culture medium powder. The method comprises wetting a dry powder cell culture medium with a solvent and then re-drying the moistened medium to obtain a dry granulated cell culture medium. Preferably, the dry granulated medium is a medium resulting from roller compaction of a dry powder medium. The term dry as used herein simply refers to the gross appearance of the granulated material and is not intended to mean that the material is completely free of complexed or agglomerated solvent unless otherwise indicated.

For use in cell culture, i.e. for culturing the cells, a liquid cell culture medium is added to the cells. A dry powder or dry compacted cell culture medium is thus dissolved in a suitable amount of liquid like water or an aqueous buffer, to generate a liquid cell culture medium that can be contacted with the cells. As the composition of the liquid cell culture medium is what directly influences the cells, the concentrations of cell culture media are often provided in weight per liter, like mg per liter, defining the concentration of a component in the liquid medium to be added to the cells. The amount of the ingredients of a dry powder or dry compacted medium need to be adjusted such that when dissolved in a certain amount of liquid the aimed concentration of the components in the resulting liquid medium is reached.

Cells to be cultured with the media according to the present invention may be prokaryotic cells like bacterial cells or eukaryotic cells like plant or animal cells. The cells can be normal cells, immortalized cells, diseased cells, transformed cells, muta nt cells, somatic cells, germ cells, stem cells, precursor cells, or embryonic cells, any of which may be established or transformed cell lines or obtained from natural sources.

Average molecular weight according to pharmacopeia is determined by titration using a phthalic anhydride-pyridine solution.

Average molecular weight determined by SEC is determined as follows:
weight average molecular weight: M_{w} = Σᵢ Nᵢ Mᵢ² / (Σᵢ Nᵢ Mᵢ)
number average molecular weight: Mₙ = Σᵢ Nᵢ Mᵢ / (Σᵢ Nᵢ)
peak molecular weight: Mₚ = molecular weight at maximum Nᵢ
Nᵢ = number of polymer species in fraction i
Mᵢ = molecular weight of polymer species in fraction i

### SEC conditions:

Calibration standards: PEG (details see Examples, Method 1)
Eluent: THF
Flow rate: 1 ml/min
Injection volume: 100 µl
Column: particle size = 5 µm, material = styrene-divinylbenzene
Temperature: 40 °C

Cell culture media can be in the form of aqueous liquids or in the form of dry powders which for use are dissolved in water or an aqueous buffer.

A person skilled in the art is able to choose a suitable cell culture medium for the specific envisaged purpose.

The cell culture media, especially the full media which comprises all components necessary to maintain and/or support the *in vitro* growth of cells, according to the present invention typically comprise at least one or more saccharide components, one or more amino acids, one or more vitamins or vitamin precursors, one or more salts, one or more buffer components, one or more co-factors, and one or more nucleic acid components. They may also additionally comprise chemically defined biochemicals such as recombinant proteins, e.g. rinsulin, rBSA, rTransferrin, rCytokines etc.

Saccharide components are all mono- or di-saccharides, like glucose, galactose, ribose, or fructose (examples of monosaccharides) or sucrose, lactose or maltose (examples of disaccharides).

Examples of amino acids according to the invention are tyrosine, the proteinogenic amino acids, especially the essential amino acids, leucine, isoleucine, lysine, methionine, phenylalanine, arginine, threonine, tryptophane, and valine, as well as the non-proteinogenic amino acids like D-amino acids, whereby the L-amino acids are preferred.

The term of the amino acids further includes the salts of the amino acids, like the sodium salts, or the respective hydrates or hydrochlorides.

For example, tyrosine means L- or D- tyrosine, preferably L-tyrosine, as well as its salts or hydrates or hydrochlorides.

Examples of vitamins are Vitamin A (Retinol, retinal, various retinoids, and four carotenoids), Vitamin B₁ (Thiamine), Vitamin B₂ (Riboflavin), Vitamin B₃ (Niacin, niacinamide), Vitamin B₅ (Pantothenic acid), Vitamin B₆ (Pyridoxine, pyridoxamine, pyridoxal), Vitamin B₇ (Biotin), Vitamin B₉ (Folic acid, folinic acid), Vitamin B₁₂ (Cyanocobalamin, hydroxycobalamin, methylcobalamin), Vitamin C (Ascorbic acid), Vitamin D (Ergocalciferol, cholecalciferol), Vitamin E (Tocopherols, tocotrienols) and Vitamin K (phylloquinone, menaquinones). Vitamin precursors are also included.

Examples of salts are components comprising inorganic ions such as bicarbonate, calcium, chloride, magnesium, phosphate, potassium, and sodium or trace elements such as Co, Cu, F, Fe, Mn, Mo, Ni, Se, Si, Ni, Bi, V and Zn. Examples are copper(II) sulphate pentahydrate (CuSO₄·5H₂O), sodium chloride (NaCl), calcium chloride (CaCl₂·2H₂O), potassium chloride (KCI), iron(II) sulphate, ferric ammonium citrate (FAC), sodium phosphate monobasic anhydrous (NaH₂PO₄), magnesium sulphate anhydrous (MgSO₄), sodium phosphate dibasic anhydrous (Na₂HPO₄), magnesium chloride hexahydrate (MgCl₂·6H₂O), zinc sulphate heptahydrate.

Examples of buffers are CO₂/HCO₃ (carbonate), phosphate, HEPES, PIPES, ACES, BES, TES, MOPS, and TRIS.

Examples of cofactors are thiamine derivatives, biotin, vitamin C, NAD/NADP, cobalamin, flavin mononucleotide and derivatives, glutathione, heme nucleotide phosphates, and derivatives.

Nucleic acid components, according to the present invention, are the nucleobases, like cytosine, guanine, adenine, thymine, or uracil, the nucleosides like cytidine, uridine, adenosine, guanosine and thymidine, and the nucleotides like adenosine monophosphate or adenosine diphosphate or adenosine triphosphate.

Feed media may have a different composition compared to full media. They typically comprise amino acids, trace elements, and vitamins. They might also comprise saccharide components but sometimes for production reasons the saccharide components are added in a separate feed.

Many biopharmaceutical production platforms are based on fed-batch cell culture protocols. The aim typically is to develop high-titer cell culture processes to meet increasing market demands and reduce manufacturing costs. Beside the use of high-performing recombinant cell lines, improvements in cell culture media and process parameters are required to realize the maximum production potentials.

In a fed-batch process, a basal medium supports initial growth and production, and a feed medium prevents the depletion of nutrients and sustains the production phase. The media are chosen to accommodate the distinct metabolic requirements during different production phases. Process parameter settings - including feeding strategy and control parameters - define the chemical and physical environments suitable for cell growth and protein production.

In a perfusion process, the cell culture medium is continuously added and removed from the bioreactors through pumps while the cells are retained in the bioreactor through a cell retention device. Advantages of perfusion are the possibility to reach very high cell densities (due to the constant medium exchange) and the possibility to produce very fragile recombinant proteins since the product can be removed from the bioreactor every day thus reducing the exposure time of the recombinant protein to high temperatures, oxidizing redox potentials or released cellular enzymes.

A process for perfusion cell culture typically comprises culturing cells in a bioreactor system comprising a bioreactor with a media inlet and a harvest outlet whereby
i. continuously or one or several times, preferably continuously, during the cell culture process new cell culture medium is inserted into the bioreactor via the media inlet
ii. continuously or one or several times during the cell culture process, preferably continuously, the harvest is removed from the bioreactor via the harvest outlet. The harvest typically comprises the target product produced by the cells, cells and liquid cell culture medium.

Poloxamers are amphiphilic polymers, with two hydrophilic blocks and a hydrophobic block in the middle. A poloxamer is a polyethylene glycol (PEG)/polypropylene glycol (PPG) tri-block copolymer whereby one PPG block is flanked on both sides with a PEG block. The polyethylene glycol (PEG) part is often also called polyethylene oxide (PEO) part. The polypropylene glycol (PPG) part is often also called the polypropylene oxide (PPO) part.

The poloxamer, CAS number 9003-11-6, which is typically used in cell culture applications is called poloxamer 188 and has the general formula I 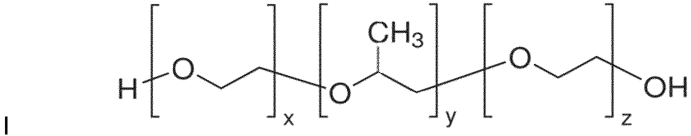 with x and z preferably independently being 75 to 85 and y preferably being 25 to 30. The poloxamers of the present invention can be found in the region with x = z = 3 to 72 and y = 5 to 12.

Some poloxamers are commercially available (Pluronic^{®} or Lutrol^{®}, e.g., a Pluronic^{®} solution, gel, or solid, such as Pluronic^{®} F-68).

Alternatively, poloxamers can be made from raw materials according to methods known in the art (see, for example, U. S. Patent Nos. 3,579,465 and 3,740,421).

Further information about poloxamers can be found in Hagers Handbuch der Pharmazeutischen Praxis, volume 9 "Stoffe P-Z", 1994, pages 282 to 284.

The following Table 1 shows examples of poloxamers.

All listed poloxamers are characterized by determining their percentage of ethylene oxide (%EO) and their molecular weight distribution. %EO was determined using ¹H NMR. The molecular weight distribution was determined with size exclusion chromatography (see Examples, Method 1). The peak molecular weight (Mₚ) was used to characterize each polymer. The molecular weight of the PPO block (MW PPO) was calculated from Mₚ and %EO.

**Table 1**

| **Poloxamer** | **%EO** | **Mₚ (g/mol)** | **MW PPO (g/mol)** |
|---|---|---|---|
| Poloxamer 99 | 89.2 | 8737 | 944 |
| Poloxamer 79a | 88.7 | 5854 | 661 |
| Poloxamer 49 | 88.4 | 3040 | 353 |
| Poloxamer 69 | 87.9 | 5177 | 626 |
| Poloxamer 79b | 87.8 | 5406 | 659 |
| Poloxamer 129 | 86.9 | 9444 | 1237 |
| Poloxamer 128 | 83.3 | 7267 | 1214 |
| Poloxamer 108 | 82.7 | 5728 | 991 |
| Poloxamer 78 | 80.6 | 3807 | 738 |
| Poloxamer 138 | 77.3 | 5756 | 1307 |
| Poloxamer 37 | 65.4 | 944 | 327 |
| Poloxamer 126 | 64.0 | 3472 | 1250 |
| Poloxamer 46 | 63.3 | 996 | 366 |
| Poloxamer 66 | 56.7 | 1354 | 586 |
| Poloxamer 75 | 53.6 | 1460 | 677 |
| Poloxamer 65 | 53.2 | 1278 | 598 |
| Poloxamer 55 | 48.1 | 1012 | 525 |
| Poloxamer 188 | 81.7 | 8300 | 1519 |
| Poloxamer 105 | 50.6 | 1960 | 968 |
| Poloxamer 184 | 38.8 | 2923 | 1789 |
| Poloxamer 123 | 34.2 | 1866 | 1228 |
| Poloxamer 282 | 25.2 | 3972 | 2971 |
| Poloxamer 101 | 15.2 | 1085 | 920 |

### Detailed description of the invention

The gist of the present invention is the finding that certain poloxamers show improved properties for use in cell culture. They do not only provide shear protection like Poloxamer 188 but also show reduced foam formation. Suitable poloxamers are also non-toxic for the cells that are cultured.

Poloxamer 188 is often added to a cell culture to prevent hydrodynamic stress caused by sparging and/or agitation. It reduces surface tension but also typically increases foam formation. As cells tend to adhere to the surface of bubbles, they rise with the bubbles to the surface area, get trapped in the foam layer, and die. It has now been found that using other poloxamers with a different composition, the positive, shear stress protecting properties can be kept but in addition, the foam formation is reduced.

Consequently, the amount of an antifoaming agent to reduce foam formation like antifoam C can be reduced or even omitted when using the cell culture media according to the present invention. In a preferred embodiment, the media according to the present invention contain less, preferably at least 25% (w/w) less, most preferred less than half of the amount of an antifoaming agent like antifoam C that is used in an equivalent cell culture under the same conditions and with the same medium except that it does not comprise a poloxamer as defined in the present invention but poloxamer 188. Examples of antifoaming agents are preferably agents comprising polydimethylsiloxane (PDMS) as well as optionally silica particles, also called e.g. dimethicone or simethicone, like Dow-Corning Q7-2587 (O/W Emulsion with 30,4% PDMS, 1.2-2.1% SiO₂ particles), Antifoam C from Sigma Aldrich (O/W Emulsion 29.4% PDMS, 1.2-2-1% SiO₂ particles) and Foam Away from Gibco (30% Simethicone emulsion in water).

The poloxamers that have been identified to be especially suitable are poloxamers with a polypropylene oxide block <700 g/mol and an ethylene oxide percentage of >45%, typically between 45% and 95%, preferably between 45% and 90%, most preferred between 75 and 90% (all w/w)..

In a preferred embodiment, the molecular weight of the PPO block is between 300 and 700 g/mol.

In a very preferred embodiment, the ethylene oxide percentage is between 75-90% (w/w) and the molecular weight of the PPO block is between 300 and 700 g/mol.

A person skilled in the art knows how to use poloxamers as ingredients in cell culture media. He is aware of the suitable amount and format to use. Typically, the poloxamer is applied to the cell culture as part of the cell culture medium. But it can also be applied separately.

The poloxamer is typically present in form of either a solid, e.g. particles, or a liquid, e.g. oil or highly viscous liquid behaving like a paste, or an aqueous solution. Preferably it is present in the form of solid particles. If its particle size needs to be adjusted, it can optionally be milled prior to adding it to the cell culture medium.

The cell culture medium according to the present invention is a cell culture medium comprising a poloxamer with a polypropylene oxide block <700 g/mol and an ethylene oxide percentage of >45%.

In a preferred embodiment, the molecular weight of the PPO block is between 300 and 700 g/mol.

In a very preferred embodiment, the ethylene oxide percentage is between 75-90% (w/w) and the molecular weight of the PPO block is between 300 and 700 g/mol.

In another preferred embodiment, the cell culture medium does not comprise an antifoaming agent or comprises a reduced amount of antifoaming agent, preferably less than 50% compared to a cell culture that does not comprise the poloxamers of the present invention but poloxamer 188.

The concentration of the poloxamer in the cell culture medium is between 0.1 to 10 g/L calculated for the liquid medium. In a very preferred embodiment, the amount of poloxamer is between 0.5 to 2 g/L calculated for the liquid medium. The poloxamer can be one type of poloxamer as defined above or a mixture of two or more of the poloxamers as defined above.

Preferably the cell culture medium is a dry powder or dry granulated medium or a liquid medium. In case of a dry medium, the medium is dissolved in a suitable amount of water or an aqueous buffer prior to use.

The cell culture medium of the present invention can be used for any type of cell culture. A cell culture is any setup in which cells are cultured.

A cell culture can be performed in any container suitable for the culture of cells, such as a petri dish, contact plate, bottle, tube, well, vessel, bag, flask, and/or tank. Preferably, it is performed in a bioreactor. Typically, the container is sterilized prior to use. Culturing is typically performed by incubation of the cells in an aqueous cell culture medium under suitable conditions such as suitable temperature, osmolality, aeration, agitation, etc. which limit contamination with foreign microorganisms from the environment. A person skilled in the art is aware of suitable incubation conditions for supporting or maintaining the growth/culturing of cells.

The present invention is thus also directed to a process for culturing cells whereby the cells are cultured in a liquid medium comprising a poloxamer with a polypropylene oxide block <700 g/mol and an ethylene oxide percentage of >45%.

The cell culture can be any set up suitable for culturing cells. Preferably it is a batch, a fed-batch, or a perfusion cell culture.

Preferably the process for culturing cells comprises the following steps:
a) providing a bioreactor
b) mixing the cells to be cultured with a cell culture medium according to the present invention.
c) incubating the mixture of step b).

In one embodiment the process comprises the following steps:
a) providing a bioreactor
b) mixing the cells to be cultured with a cell culture medium according to the present invention
c) incubating the mixture of step b)
whereby a cell culture medium, which is, in this case, a feed medium, is added to the bioreactor, continuously over the whole time or once or several times within the cells incubation time of step c).

The feed medium may be a cell culture medium according to the present invention, but it may also be a feed medium that does not comprise a poloxamer. Preferably it does not comprise a poloxamer.

**In** one embodiment, the bioreactor is a perfusion bioreactor. A perfusion bioreactor is a bioreactor in which perfusion cell culture can be performed. **It** comprises the bioreactor vessel which is typically closed during cell culture, a stirrer in the vessel, a line for introducing fresh medium, a harvest line for removing the harvest stream comprising cells, liquid medium and target product from the bioreactor and a cell retention device in the harvest line that retains the cells while the liquid part of the harvest can be collected. A review about perfusion cell culture providing details about favorable set ups can be found in "Perfusion mammalian cell culture for recombinant protein manufacturing - A critical review" Jean-Marc Bielser et al., Biotechnology Advances 36 (2018) 1328-1340.

**In** a perfusion process, the cell culture medium is continuously added and removed from the bioreactors through pumps while the cells are retained in the bioreactor through a cell retention device. Advantages of perfusion is the possibility to reach very high cell densities (due to the constant medium exchange) and the possibility to produce very fragile recombinant proteins since the product can be removed from the bioreactor every day thus reducing the exposure time of the recombinant protein to high temperatures, oxidizing redox potentials or released cellular proteases.

**In** one embodiment, the process of the present invention comprises culturing cells in a bioreactor system comprising a bioreactor with a media inlet and a harvest outlet whereby
i. continuously or one or several times, preferably continuously, during the cell culture process new cell culture medium according to the present invention is inserted into the bioreactor via the media inlet
ii. continuously or one or several times during the cell culture process, preferably continuously, harvest is removed from the bioreactor via the harvest outlet. The harvest typically comprises the target product produced by the cells, cells and liquid cell culture medium.

When using a cell culture medium according to the present invention the culture shows equal performance and reduced foam formation compared to a cell culture that is performed under the same conditions but with a medium comprising poloxamer 188 instead of the poloxamers as defined above.

The present invention is therefore also directed to a method for reducing foam formation in agitated and/or sparged cell cultures whereby the cells are cultured in a liquid medium comprising a poloxamer with a polypropylene oxide block <700 g/mol and an ethylene oxide percentage of >45% and the foam formation is reduced compared to cells cultured under the same conditions in a cell culture medium that comprises poloxamer 188 instead of a poloxamer with a polypropylene oxide block <700 g/mol and an ethylene oxide percentage of >45%.

The present invention is also directed to a method for culturing cells in agitated and/or sparged cell cultures whereby the cells are cultured in a liquid medium comprising a poloxamer with a polypropylene oxide block <700 g/mol and an ethylene oxide percentage of >45% and the amount of an antifoaming agent in the liquid medium is reduced compared to cells cultured under the same conditions in a cell culture medium that comprises poloxamer 188 instead of a poloxamer with a polypropylene oxide block <700 g/mol and an ethylene oxide percentage of >45%. Preferably the amount of antifoaming agent is reduced by at least 25%, preferably by at least 50%.

### Examples

### Method 1

All size exclusion chromatography (SEC) measurements are carried out as follows:
Calibration standards: PEG (Mp: 430, 982, 1960, 3020, 6690, 12300, 26100 and 44 000 g/mol)
Eluent: THF
Flow rate: 1 mL/min
Injection volume: 100 µL
Column: particle size = 5 µm, material = styrene-divinylbenzene
Temperature: 40 °C
Detector: refractive index (RI)

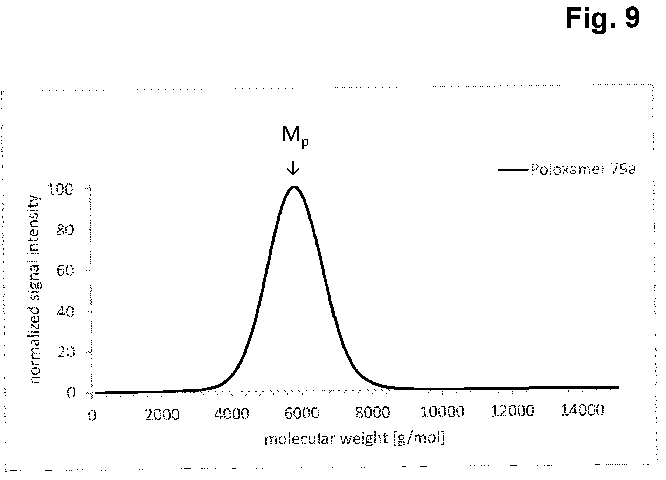

Molecular weight distribution curves were calculated for all poloxamers and Mp (peak maximum) was determined (Table 1). An exemplary distribution curve is shown in Figure 9 which shows the Molecular weight distribution determined with size exclusion chromatography for poloxamer 79a. Mp indicates the peak molecular weight of the specific poloxamer.

### Method 2:

### Foam Evolution measurements of Poloxamer in water at 37°C

### Preparation of the poloxamer solution in water

A solution of the target poloxamer in MilliQ-water was prepared at the selected concentration and then stirred or mixed via a roller mixer overnight until complete dispersion. The solutions were prepared by weight.

### Foam measurements

The foam evolution was tested by using the Dynamic Foam Analyzer DFA 100 (Krüss GmbH).

The following parameters were applied:
Flow rate (air): 0.3 L/min
Temperature: 37 °C
Liquid volume: 15 mL
Duration of measurement: 60 min
Height illumination: 20 %
Min. bubble area: 1200 µm²
Max. bubble area: 35 mm²
Min. histogram area: 0 mm²
Max. histogram area: 1 mm²

The poloxamer solution was poured into the glass column of the Foam Analyzer DFA 100. The solution was heated to 37 °C because this is the temperature employed in bioreactors. The gas flow was started and the foam formation (foam height, liquid height, and foam structure) was recorded by the Foam Analyzer over a time frame of 60 minutes. In case the foam reached the maximum column height of 210 mm, the gas flow automatically stopped to prevent overflow.

### Method 3

### Toxicity assay

To determine the toxic properties of the poloxamers, the following cell culture toxicity assay was performed with two in-house cell lines - CHO-DG44 and CHOZN. The two cell lines were passaged at least until passage 5 before being tested in the experiment. Cellvento^{®} CHO-220 comp. (1.03687 | Merck KGaA; Darmstadt, Germany) a chemically defined cell culture medium containing 2 g/L Poloxamer 188 was used as basis medium for the toxicity assay. In the case of CHO-DG44 cell line, the basis medium was supplemented with 8 mM L-Glutamine (1.00286 | CAS 56-85-9 | Merck KGaA; Darmstadt, Germany). The basis media have been prepared according to the user's guide. A stock solution of each poloxamer was prepared with a concentration of 4 g/L. The stock solutions have been dissolved for at least one hour. Dilutions with a poloxamer concentration of 1 g/L were prepared from the basic media and the stock solution. The pH was adjusted to 7.0 ±0.1 by using 25 % hydrochloric acid (1.00316 1 Merck KGaA; Darmstadt, Germany) and 32 % sodium hydroxide solution (1.05587 | CAS 1310-73-2 | Merck KGaA; Darmstadt, Germany). The osmolality was measured and adjusted to 335±15 mOsmol/kg with sodium chloride (1.06400 | CAS 7647-14-5 | Merck KGaA; Darmstadt, Germany). The media was sterilized by filtration using 0.22 µm Stericup^{®} & Steritop^{®} Filter Units (Merck KGaA; Darmstadt, Germany).

The starting VCD for each experiment was 0.5 x 10⁶ viable cells/mL in a volume of 30 mL. Therefore, an adequate volume of cell suspension (including 15 x 10⁶ viable cells) was transferred into a 50 mL TPP TubeSpin^{®} bioreactor (TPP; Trasadingen, Switzerland) and centrifuged at 1,200 rpm and 4 °C for five minutes. The supernatant was discarded, and the cell pellet was resuspended in 30 mL of specific medium. Cells were cultured for four days at 37 °C, 320 rpm, 5% CO₂, and 80% humidity. VCD and viability were measured on day 0, 1, 2, 3 and 4. Triplicates were applied per condition.

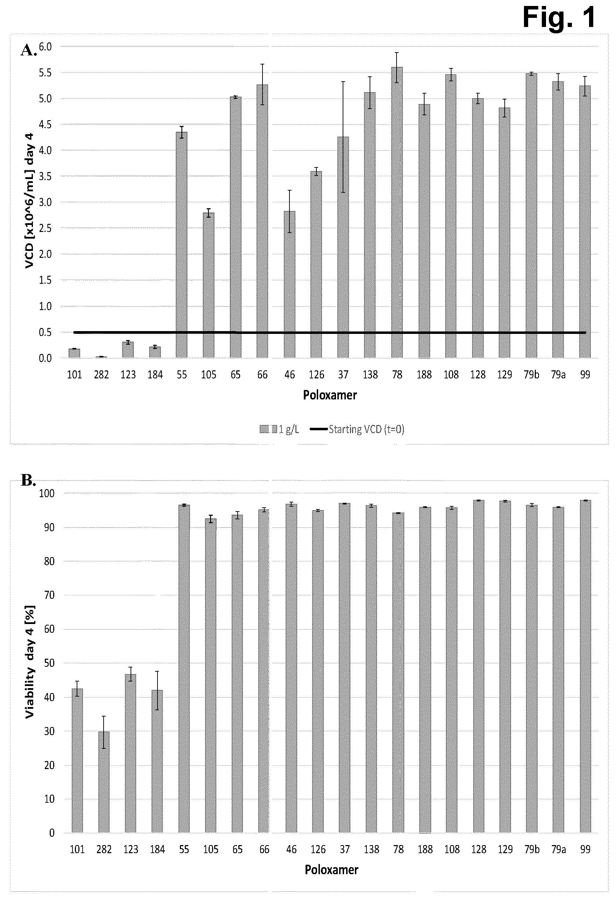

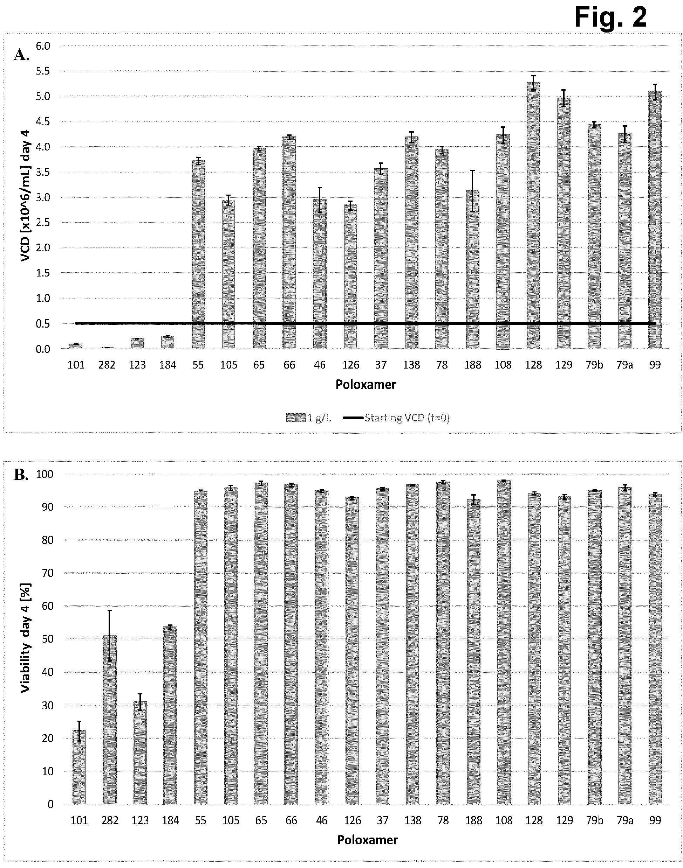

In Figure 1 and Figure 2, the results of viability are given as mean plus-minus standard deviation. Results of VCD were reported for day 4 with their standard deviation.

### Method 4

### Shear stress assay

To investigate the protective property of poloxamers at very high shear rates, the following shear stress assay was developed.

CHO-S cell line was used for this assay and passaged at least until passage 5 and maximum until passage 25 before being tested for shear protection. Cellvento CHO-220 comp. (1.03687 | Merck KGaA; Darmstadt, Germany) containing 2 g/L Poloxamer 188 and supplemented with 8 mM L-Glutamine (1.00286 | CAS 56-85-9 | Merck KGaA; Darmstadt, Germany) was used as passage medium and prepared according to the user's guide. Cellvento CHO-220 (1.03797 | Merck KGaA; Darmstadt, Germany) without Poloxamer 188 and supplemented with 6 mM L-Glutamine (1.00286 | CAS 56-85-9 | Merck KGaA; Darmstadt, Germany) has been used as medium basis. The basis media have been prepared according to the user's guide. Poloxamer 188 EMPROVE^{®} EXPERT cell culture optimized (1.37097 | LOT 48854497711 | CAS 9003-11-6 | Merck KGaA, Darmstadt, Germany) in a concentration of 1 g/L has been used as positive control. As negative control (NC), a mixture of P188 EMPROVE and P407 (16758 | LOT BCBV0465 | CAS 9003-11-6 | Sigma-Aldrich, St. Louis, U.S.A.) at a concentration of 1 g/L and a ratio 99:1 (m%) was used. A stock solution of 1 g/L of each poloxamer has been produced. After weighing, and adding to the medium basis, the stock solutions and controls have been dissolved for at least one hour. Afterward, the pH was adjusted to 7.0 ±0.1 by using 25 % hydrochloric acid (1.00316 | Merck KGaA; Darmstadt, Germany) and 32 % sodium hydroxide solution (1.05587 | CAS 1310-73-2 | Merck KGaA; Darmstadt, Germany). The osmolality was measured and if necessary, adjusted with sodium chloride (1.06400 | CAS 7647-14-5 | Merck KGaA; Darmstadt, Germany) to 335 ±15 mOsmol/kg. Finally, the media were sterilized by filtration using 0.22 µm Stericup^{®} and Steritop^{®} Filter Units (Merck KGaA; Darmstadt, Germany).

250 mL baffled shake flasks (Corning; New York, U.S.A.) containing a total volume of 50 mL were used as test vessels at a high shaking speed of 350 rpm to generate shear stress. 46 mL of specific medium was prefilled in baffled shake flasks. Each experiment was performed in triplicates.

The shear stress assay was starting with a VCD of 1.5 x 10⁶ viable cells/mL. Therefore, the required volume of cell suspension including 75 x 10⁶ viable cells was transferred into 50 mL TPP TubeSpin^{®} bioreactors (TPP; Trasadingen, Switzerland), and centrifuged at 1,200 rpm and 4 °C for five minutes. The supernatant was discarded, and the cell pellet was resuspended in 4 mL of specific medium and after all added to the 46 mL specific medium which has been prefilled in the baffled shake flasks. Samples were taken immediately at time t = 0. VCD and viability were measured subsequently. The baffled shake flasks were incubated for 4 hours at 5% CO₂, 37 °C and 350 rpm. VCD and viability were measured every hour of shaking for four hours.

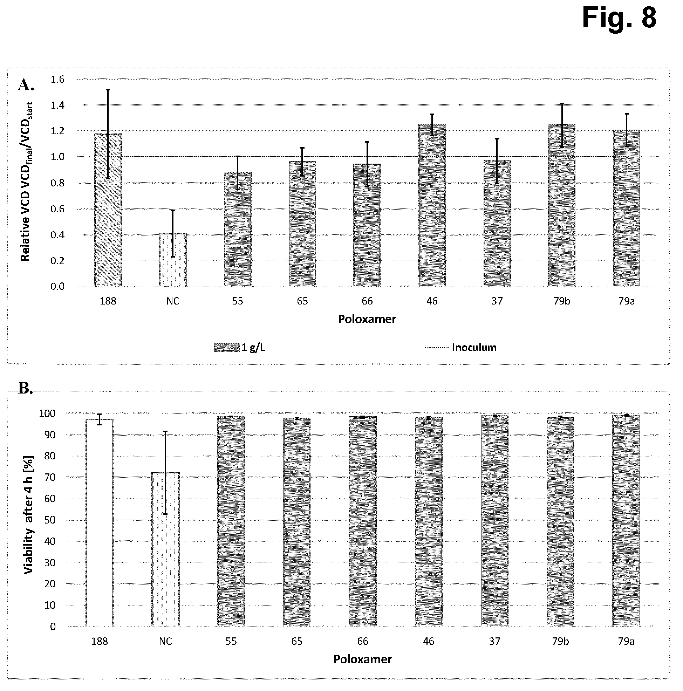

In Figure 8, the results of viability are given as mean plus minus standard deviation. Results of relative VCD (rVCD) were calculated as a ratio of VCD_{final} and VCDₛₜₐᵣₜ and the gaussian propagation of error.

### Method 5

### Fed-batch cell culture

Fed-batch cell culture was performed to evaluate the protective performances of the poloxamer during the culture. CHOK1SVD1 cells were employed. The culture was carried out in spin tubes (TPP, Art. No. 87050) with a final volume of 30 mL at 37°C and 320 rpm rotation speed.

Before the start of the fed-batch, the cells were cultivated in the cell culture medium 4CHO (Merck KGaA, 103795) containing 2 g/L Poloxamer 188. To this medium HT-supplements 100x (sodium-hypoxanthine (10 mM) and thymidine (1,6 mM), Gibco^{®}, Life technologies, Art. No. 11067-030) and L-Methionine sulfoximine (Sigma Aldrich M5379) were added.

On day 0 of the fed-batch, the cells were centrifuged for 5 min at 2000 rpm, then resuspended in 4CHO+HT without poloxamer 188 and pooled.

The CHOK1 SVD1 cells were seeded in different conditions (4 replicates each) with 0.2x10⁶ cells per mL. For each conditions the cell culture medium 4CHO without poloxamer 188 (Merck KGaA, 4.74000.9999) was employed and the selected poloxamers were added to achieve the concentration of 1 g/L. The solutions were stirred for at least two hours to allow complete dispersion.

The cells were fed on days 3, 5, 7 and 10.

Glucose concentration was measured daily starting from D3. A continuous feeding of 6 g/L glucose on demand, was maintained during the whole fermentation process.

Analysis of VCD and Viability with the ViCell (Beckman Coulter) and determination of IgG by using the Cedex (Bio HT Analyzer, Roche) were performed daily.

| Step | Description |
|---|---|
| Pre culture (seed train strategy) | 6x30 mL pre-culture (CHOK1SVD1) in 4CHO medium, incubated at 37 °C, 5% CO₂ and 320 rpm. Spin tubes were pooled on D0 before inoculation of the main culture. |
| Main culture | 30mL spin tubes as fed-batch using a seeding density of 0.2x10⁶ viable cells mL⁻¹ in 4CHO medium (including different poloxamers) incubated at 37 °C, 320 rpm for 13 days. |
| Feeding strategy | D3 (2.75%), D5 (2.75%), D7 (4.1%), D10 (4.1%), D13 (2.75%) |

In Figure 11, the results of VCD, viability and IgG productivity are given as mean value of the 4 replicates of each condition and the error bars are the standard deviation of the average values.

### Example 1 Toxicity

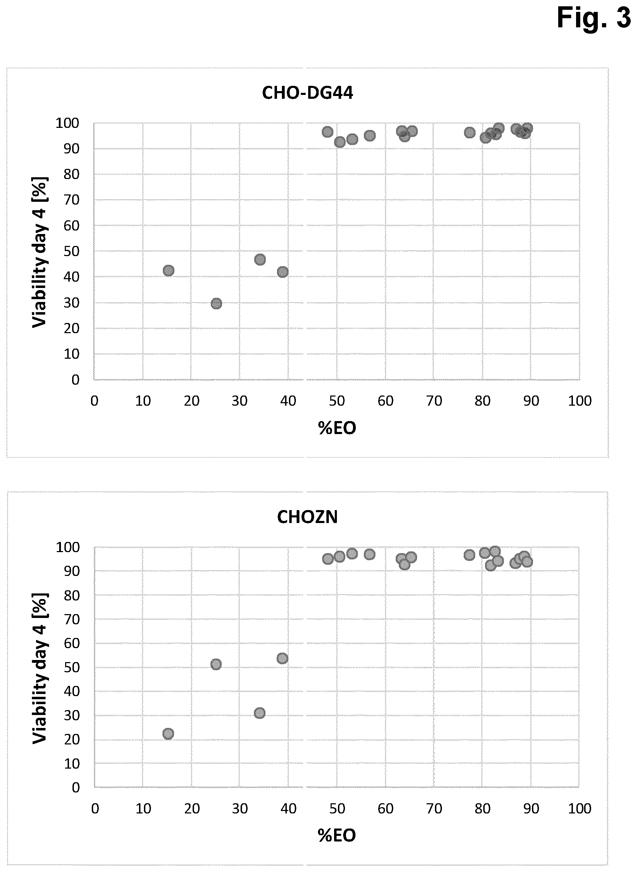

The toxic effect on CHO cells was investigated for the poloxamers described in Table 1. The toxicity cell assay is described in Method 3. Poloxamers with less than 45%EO are found to be toxic for both cell lines CHO-DG44 and CHOZN (Figures 1 and 2). Their addition to the cell culture results in a significant decrease in cell viability. The data show that viability clearly correlates with %EO. Poloxamers with %EO <45 result in low viabilities and are thus toxic for the CHO cells (Figure 3). In contrast, the addition of poloxamers with %EO >45 results in viabilities >90%. Those samples are therefore non-toxic.

Figure 1: Toxicity assay of poloxamers on CHO-DG44 cell line. The Poloxamers are reported with an increasing percentage of EO from left to right. Poloxamers were tested in a concentration of 1 g/L (grey bars) for four days (replicates n = 3). **A.** VCD of CHO-DG44 on day four. The black line represents the starting VCD (t=0) of 0.5 x 10⁶ viable cells/mL.

**B.** Viability of CHO-DG44 on day four.

Figure 2 Toxicity assay of poloxamers on CHOZN cell line. The Poloxamers are reported with an increasing percentage of EO from left to right.

Poloxamers were tested in a concentration of 1 g/L (grey bars) for four days (replicates n = 3). A. VCD of CHOZN on day four. The black line represents the starting VCD (t=0) of 0.5 x 10⁶ viable cells/mL. B. Viability of CHOZN on day four.

Figure 3: Correlation between cell viability and %EO in poloxamers.

### Example 2: Foaming

The foaming behavior of all non-toxic poloxamers was investigated. The experimental setup is described in Method 2.

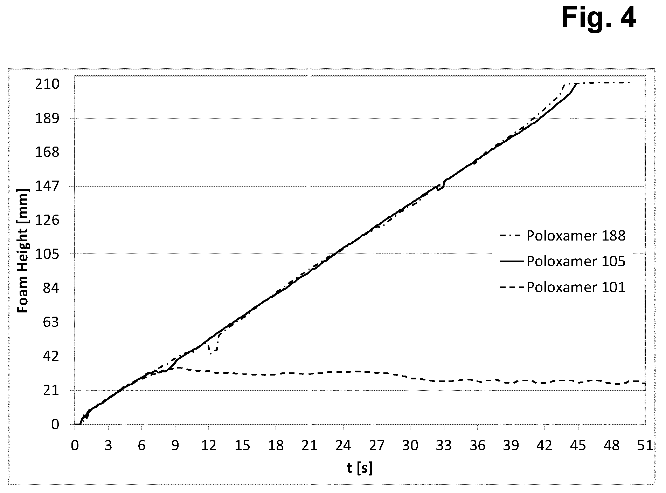

The foaming behavior of Poloxamer 188 at 0.25 g/L (i.e., 250 ppm) concentration in water is reported in Figure 4. Within 50 s the maximum foam height of 210 mm (i.e., length of the foam analyzer column) is reached, and the measurement is automatically stopped by the instrument to prevent overflow.

Poloxamer 101 is selected as negative control and, as expected, only very little foam is created upon sparging (Figure 4) and the foam immediately disappears after sparging stops (data not shown). This behavior is in clear contrast to Poloxamer 188.

Poloxamer 105 was expected to generate more foam than Poloxamer 101 but less foam than Poloxamer 188, because of its intermediate hydrophobicity (50% EO). Surprisingly, the foaming behavior of Poloxamer 105 turned out to be very similar to Poloxamer 188. Maximum foam height was reached after 44 seconds (Figure 4).

Foam evolution during continuous sparging at 37°C for the poloxamers 99, 129, 128, 108, 78, 138, and 126 show similar foaming behavior to Poloxamer 188 and 105. After 44-50 seconds maximum foam height of 210 mm was reached and sparging stopped. Differences were only observed in the foam decay, but foam decay is considered not relevant in bioreactors because the process is carried out under continuous sparging.

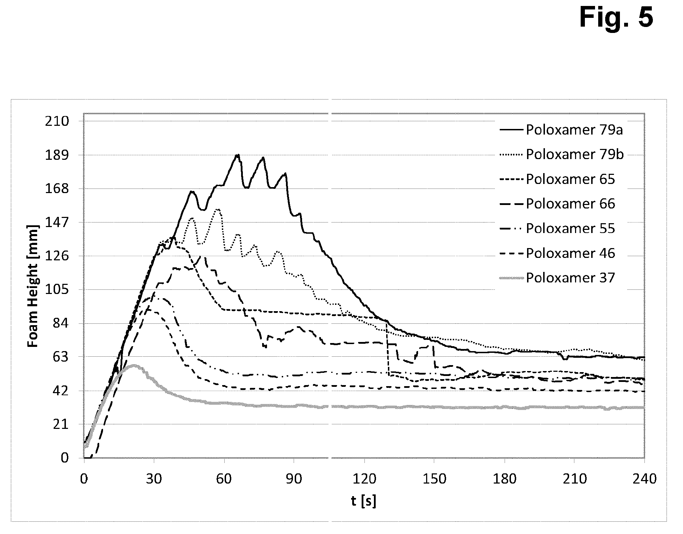

In the case of poloxamers 79a, 79b, 66, 65, 55, 46, and 37 foam heights between 190 and 60 mm were reached for a short period of time, but then the foam height decreased quickly and it reached a constant height of approx. 30 -70 mm (Figure 5, Table). The decrease of the foam height and the formation of a constant foam level, namely plateau height, can be explained in terms of an equilibrium between foam formation due to sparging and foam decay due to drainage and bubble coalescence.

Figure 5: Foam evolution during continuous sparging at 37°C of poloxamers with different hydrophobicity and molecular weights (water solutions 250 ppm). The plots display maximum foam and plateau heights. The curves reported are one measurement selected out of the replicates as representative of the trend for each poloxamer.

**Table 2: Maximum foam height and plateau height. 0.25 g/L (250 ppm) poloxamer solutions in water, 37°C.**

| **Poloxamer** | **Average Max. Foam Height [mm]** | **Average Plateau Height [mm]** |
|---|---|---|
| Poloxamer 79a | 189 | 68 |
| Poloxamer 79b | 150 | 57 |
| Poloxamer 65 | 130 | 36 |
| Poloxamer 66 | 130 | 43 |
| Poloxamer 55 | 120 | 46 |
| Poloxamer 46 | 90 | 39 |
| Poloxamer 37 | 70 | 32 |

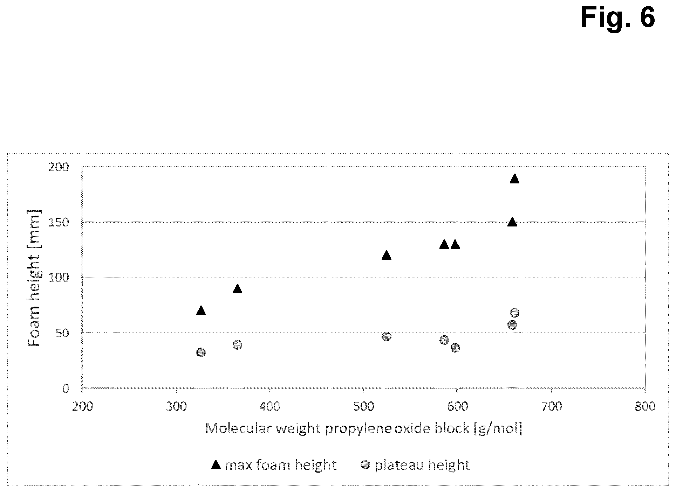

In contrast to what is known from literature, it turns out that PPO block length is the critical parameter influencing foaming behavior. The molecular weight of the PPO block correlates with the maximum foam height (Figures 5 and 6, Table 2). The higher the molecular weight of the PPO block, the higher the maximum foam height is.

Figure 6: Correlation between average maximum foam height as well as average plateau height and molecular weight of the polypropylene oxide block in poloxamers.

Low foaming was observed for all poloxamers with a PPO molecular weight <700 g/mol, while %EO may vary between 45% and 95% and in this range has little impact on foaming behavior.

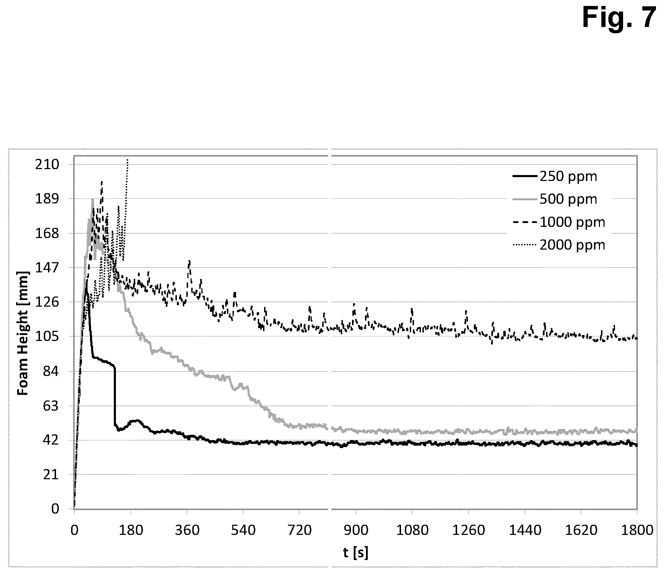

A higher poloxamer concentration results in more foam being generated. This phenomenon is shown in Figure 7 for poloxamer 65 as an example. The foaming behavior of poloxamer 65 was tested at four different concentrations: 250, 500, 1000, and 2000 ppm. Maximum foam height and plateau height increased with increasing poloxamer concentration. This evidence shows that it is important to compare the foaming behavior of different poloxamers at the same concentration. For all the above measurements a concentration of 250 ppm was chosen, to be able to observe differences in foaming behavior with the Dynamic Foam Analyzer DFA 100 and prevent column overflow. The foaming trends observed at 250 ppm are comparable to those observed at higher concentrations.

Figure 7: Foam evolution of Poloxamer 65 water solutions at 37 °C. Different concentrations were tested.

### Example 3: shear stress protection

In the shear stress protection assay (see Method 4), all non-toxic and low-foaming poloxamers were tested and compared to the benchmark Poloxamer 188 (Figure 8). All poloxamers, except the negative control, provide sufficient shear stress protection for CHO cells similar to Poloxamer 188 (Figure 8A). Very good viabilities of ≥90% are observed for all poloxamers tested except for the negative control (Figure 8B).

Figure 8: Shear stress assay of poloxamers on CHO-S cell line. The Poloxamers are reported with an increasing percentage of EO from left to right. Poloxamers were tested with a concentration of 1 g/L for four hours (replicates n = 3). The positive control 1 g/L Poloxamer 188 is shown on the left side (diagonal stripes, n = 32). As a negative control (NC) P188 and P407 at a concentration of 1 g/L and the ratio of 99:1 (wt.%) was used (vertical stripes, n = 32). **A.** Relative VCD: The relative VCD of CHO-S was calculated as ratio VCD_{final}/VCDₛₜₐᵣₜ. The dotted line represents the relative inoculum VCD (t=0). **B.** Viability (%) of CHO-S after four hours.

### Example 4: fed-batch cell culture

Fed-batch cell culture (see Method 5) was performed to investigate the shear stress protection properties of 3 different poloxamers, namely 75, 69, and 49 compared to the benchmark 188.

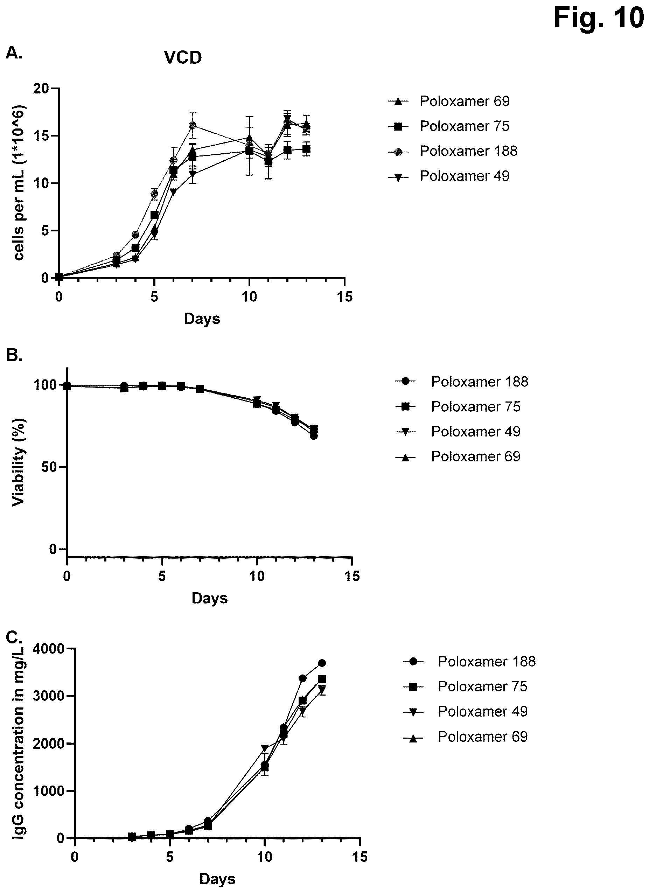

These poloxamers are low foaming and non-toxic like the other poloxamers of similar molecular weight and ethoxylation degree. Poloxamer 75 is liquid and has an ethoxylation degree of 53.6, instead poloxamer 69 and 49 are solid and more hydrophilic, having a %EO around 88%. Poloxamer 49 has also a lower molecular weight compared to the other poloxamers with comparable hydrophilicity. Being solid and low foaming poloxamer 69 and 49 are particularly appealing for the implementation in cell culture media. The viable cell densities, viabilities and IgG productivity (Figure 10) are similar for all four conditions. The three custom-made poloxamers have comparable performances to poloxamer 188 and are confirmed to be sufficiently shear stress protectors to support the cell culture and the antibody production.

Figure 10. Fed-batch cell culture results. The culture was carried out for 13 days in spin tubes. All the poloxamer solutions in 4CHO cell culture medium have concentration of 1 g/L. **A.** Viable cell density (VCD) of CHOK1 SVD1 cells during the fed-batch culture. Each value is the average of the 4 replicates of each condition. Error bars are the standard deviation of the average. **B.** Viability (%) of CHOK1 SVD1 cells during the fed-batch culture. Each value is the average of the 4 replicates of each condition. Error bars are the standard deviation of the average. **C.** IgG productivity of CHOK1 SVD1 cells during the fed-batch culture. Each value is the average of the 4 replicates of each condition. Error bars are the standard deviation of the average.

## Claims

1. A cell culture medium comprising a poloxamer with a polypropylene oxide block ≤700 g/mol and an ethylene oxide percentage of >45%, whereby the cell culture medium comprises the poloxamer in an amount between 0.1 to 10 g/L calculated for the liquid medium.

2. A cell culture medium according to claim 1, **characterized in that** the molecular weight of the PPO block is between 300 and 700 g/mol.

3. A cell culture medium according to claim 1 or claim 2, **characterized in that** the ethylene oxide percentage is between 75% and 90% (w/w) and the molecular weight of the PPO block is between 300 and 700 g/mol.

4. A cell culture medium according to one or more of claims 1 to 3, **characterized in that** the cell culture medium is a chemically defined medium.

5. A cell culture medium according to one or more of claims 1 to 4, **characterized in that** the cell culture medium is a dry powder or a dry granulated medium.

6. A cell culture medium according to one or more of claims 1 to 5, **characterized in that** the cell culture medium comprises at least one or more saccharide components, one or more amino acids, one or more vitamins or vitamin precursors, one or more salts, one or more buffer components, one or more co-factors, and one or more nucleic acid components.

7. A process for culturing cells whereby the cells are cultured in a liquid medium comprising a poloxamer with a polypropylene oxide block ≤700 g/mol and an ethylene oxide percentage of >45%, whereby the cell culture medium comprises the poloxamer in an amount between 0.1 to 10 g/L calculated for the liquid medium.

8. A process for culturing cells according to claim 7, **characterized in that** the molecular weight of the **PPO** block is between 300 and 700 g/mol.

9. A process for culturing cells according to claim 7 or 8, **characterized in that** the ethylene oxide percentage is between 75% and 90% (w/w) and the molecular weight of the **PPO** block is between 300 and 700 g/mol.

10. A process for culturing cells according to one or more of claims 7 to 9, **characterized in that** the process includes
a) providing a bioreactor
b) mixing the cells to be cultured with a cell culture medium according to one or more of claims 1 to 6
c) incubating the mixture of step b).

11. A process for culturing cells according to one or more of claims 7 to 10, **characterized in that** the bioreactor is a perfusion bioreactor.

12. A process for culturing cells according to one or more of claims 7 to 10, **characterized in that** the process comprises the following steps:
a) providing a bioreactor
b) mixing the cells to be cultured with a cell culture medium according to one or more of claims 1 to 6
c) incubating the mixture of step b)
whereby a cell culture medium, which is, in this case, a feed medium, is added to the bioreactor, continuously over the whole time or once or several times within the cells incubation time of step c).

13. A process for culturing cells according to one or more of claims 7 to 11, **characterized in that** the amount of antifoaming agent in the liquid medium is reduced compared to a cell culture medium that is otherwise identical but comprises poloxamer 188 instead of a poloxamer with a polypropylene oxide block <700 g/mol and an ethylene oxide percentage of >45%.

14. A method for reducing foam formation in agitated and/or sparged cell cultures whereby the cells are cultured in a liquid medium comprising a poloxamer with a polypropylene oxide block ≤700 g/mol and an ethylene oxide percentage of >45% in an amount between 0.1 to 10 g/L calculated for the liquid medium and the foam formation is reduced compared to cells cultured under the same conditions in a cell culture medium that comprises poloxamer 188 instead of a poloxamer with a polypropylene oxide block ≤700 g/mol and an ethylene oxide percentage of >45%.

## Patentansprüche

1. Zellkulturmedium enthaltend ein Poloxamer mit einem Polypropylenoxidblock ≤700 g/mol und einem Prozentanteil an Ethylenoxid von >45 %, wobei das Zellkulturmedium das Poloxamer in einer Menge zwischen 0,1 und 10 g/L enthält, berechnet für das flüssige Medium.

2. Zellkulturmedium nach Anspruch 1, **dadurch gekennzeichnet, dass** das Molekulargewicht des PPO-Blocks zwischen 300 und 700 g/mol liegt.

3. Zellkulturmedium nach Anspruch 1 oder Anspruch 2, **dadurch gekennzeichnet, dass** der Prozentanteil an Ethylenoxid zwischen 75 % und 90 % (w/w) und das Molekulargewicht des PPO-Blocks zwischen 300 und 700 g/mol liegt.

4. Zellkulturmedium nach einem oder mehreren der Ansprüche 1 bis 3, **dadurch gekennzeichnet, dass** das Zellkulturmedium ein chemisch definiertes Medium ist.

5. Zellkulturmedium nach einem oder mehreren der Ansprüche 1 bis 4, **dadurch gekennzeichnet, dass** das Zellkulturmedium ein trockenes Pulver oder ein trockenes granuliertes Medium ist.

6. Zellkulturmedium nach einem oder mehreren der Ansprüche 1 bis 5, **dadurch gekennzeichnet, dass** das Zellkulturmedium mindestens eine oder mehrere Saccharidkomponenten, eine oder mehrere Aminosäuren, ein oder mehrere Vitamine oder Vitaminvorstufen, ein oder mehrere Salze, eine oder mehrere Pufferkomponenten, einen oder mehrere Cofaktoren und eine oder mehrere Nukleinsäurekomponenten enthält.

7. Verfahren zum Kultivieren von Zellen, wobei die Zellen in einem flüssigen Medium kultiviert werden, das ein Poloxamer mit einem Polypropylenoxidblock ≤700 g/mol und einem Prozentanteil an Ethylenoxid von >45 % enthält, wobei das Zellkulturmedium das Poloxamer in einer Menge zwischen 0,1 und 10 g/L enthält, berechnet für das flüssige Medium.

8. Verfahren zum Kultivieren von Zellen nach Anspruch 7, **dadurch gekennzeichnet, dass** das Molekulargewicht des PPO-Blocks zwischen 300 und 700 g/mol liegt.

9. Verfahren zum Kultivieren von Zellen nach Anspruch 7 oder 8, **dadurch gekennzeichnet, dass** der Prozentanteil an Ethylenoxid zwischen 75 % und 90 % (w/w) und das Molekulargewicht des PPO-Blocks zwischen 300 und 700 g/mol liegt.

10. Verfahren zum Kultivieren von Zellen nach einem oder mehreren der Ansprüche 7 bis 9, **dadurch gekennzeichnet, dass** das Verfahren umfasst:
a) Bereitstellen eines Bioreaktors
b) Mischen der zu kultivierenden Zellen mit einem Zellkulturmedium nach einem oder mehreren der Ansprüche 1 bis 6
c) Inkubieren der Mischung aus Schritt b).

11. Verfahren zum Kultivieren von Zellen nach einem oder mehreren der Ansprüche 7 bis 10, **dadurch gekennzeichnet, dass** der Bioreaktor ein Perfusionsbioreaktor ist.

12. Verfahren zum Kultivieren von Zellen nach einem oder mehreren der Ansprüche 7 bis 10, **dadurch gekennzeichnet, dass** das Verfahren die folgenden Schritte umfasst:
a) Bereitstellen eines Bioreaktors
b) Mischen der zu kultivierenden Zellen mit einem Zellkulturmedium nach einem oder mehreren der Ansprüche 1 bis 6
c) Inkubieren der Mischung aus Schritt b)
wobei ein Zellkulturmedium, das in diesem Fall ein Zufütterungsmedium ist, zum Bioreaktor hinzugegeben wird, kontinuierlich über den ganzen Zeitraum oder einmal oder mehrmals innerhalb des Inkubationszeitraums der Zellen in Schritt c).

13. Verfahren zum Kultivieren von Zellen nach einem oder mehreren der Ansprüche 7 bis 11, **dadurch gekennzeichnet, dass** die Menge an Antischaummittel in dem flüssigen Medium verglichen mit einem Zellkulturmedium, das ansonsten identisch ist, aber Poloxamer 188 anstelle eines Poloxamers mit einem Polypropylenoxidblock <700 g/mol und einem Prozentanteil an Ethylenoxid von >45 % enthält, reduziert ist.

14. Methode zum Reduzieren der Schaumbildung in gerührten und/oder durchperlten Zellkulturen, wobei die Zellen in einem flüssigen Medium kultiviert werden, das ein Poloxamer mit einem Polypropylenoxidblock ≤700 g/mol und einem Prozentanteil an Ethylenoxid von >45 % in einer Menge zwischen 0,1 und 10 g/L, berechnet für das flüssige Medium, enthält, und die Schaumbildung verglichen mit Zellen, die unter denselben Bedingungen in einem Zellkulturmedium, das Poloxamer 188 anstelle eines Poloxamers mit einem Polypropylenoxidblock ≤700 g/mol und einem Prozentanteil an Ethylenoxid von >45 % enthält, kultiviert werden, reduziert ist.

## Revendications

1. Milieu de culture cellulaire comprenant un poloxamère ayant un bloc d'oxyde de polypropylène ≤700 g/mol et un pourcentage d'oxyde d'éthylène >45%, le milieu de culture cellulaire comprenant le poloxamère selon une quantité comprise entre 0,1 et 10 g/L calculée pour le milieu liquide.

2. Milieu de culture cellulaire selon la revendication 1, **caractérisé en ce que** le poids moléculaire du bloc de PPO est compris entre 300 et 700 g/mol.

3. Milieu de culture cellulaire selon la revendication 1 ou la revendication 2, **caractérisé en ce que** le pourcentage d'oxyde d'éthylène est compris entre 75% et 90% (p/p) et le poids moléculaire du bloc de PPO est compris entre 300 et 700 g/mol.

4. Milieu de culture cellulaire selon l'une ou plusieurs parmi les revendications 1 à 3, **caractérisé en ce que** le milieu de culture cellulaire est un milieu défini chimiquement.

5. Milieu de culture cellulaire selon l'une ou plusieurs parmi les revendications 1 à 4, **caractérisé en ce que** le milieu de culture cellulaire est une poudre sèche ou un milieu granulé sec.

6. Milieu de culture cellulaire selon l'une ou plusieurs parmi les revendications 1 à 5, **caractérisé en ce que** le milieu de culture cellulaire comprend au moins un ou plusieurs composants saccharidiques, un ou plusieurs acides aminés, un(e) ou plusieurs vitamines ou précurseurs de vitamines, un ou plusieurs sels, un ou plusieurs composants de tampon, un ou plusieurs cofacteurs, et un ou plusieurs composants d'acide nucléique.

7. Procédé destiné à la culture de cellules, dans lequel les cellules sont cultivées dans un milieu liquide comprenant un poloxamère ayant un bloc d'oxyde de polypropylène ≤700 g/mol et un pourcentage d'oxyde d'éthylène >45%, où le milieu de culture cellulaire comprend le poloxamère selon une quantité comprise entre 0,1 et 10 g/L calculée pour le milieu liquide.

8. Procédé destiné à la culture de cellules selon la revendication 7, **caractérisé en ce que** le poids moléculaire du bloc de PPO est compris entre 300 et 700 g/mol.

9. Procédé destiné à la culture de cellules selon la revendication 7 ou 8, **caractérisé en ce que** le pourcentage d'oxyde d'éthylène est compris entre 75% et 90% (p/p) et le poids moléculaire du bloc de PPO est compris entre 300 et 700 g/mol.

10. Procédé destiné à la culture de cellules selon l'une ou plusieurs parmi les revendications 7 à 9, **caractérisé en ce que** le procédé comporte
a) la mise à disposition d'un bioréacteur
b) le mélange des cellules à cultiver avec un milieu de culture cellulaire selon l'une ou plusieurs parmi les revendications 1 à 6
c) l'incubation du mélange de l'étape b).

11. Procédé destiné à la culture de cellules selon l'une ou plusieurs parmi les revendications 7 à 10, **caractérisé en ce que** le bioréacteur est un bioréacteur à perfusion.

12. Procédé destiné à la culture de cellules selon l'une ou plusieurs parmi les revendications 7 à 10, **caractérisé en ce que** le procédé comprend les étapes suivantes :
a) la mise à disposition d'un bioréacteur
b) le mélange des cellules à cultiver avec un milieu de culture cellulaire selon l'une ou plusieurs parmi les revendications 1 à 6
c) l'incubation du mélange de l'étape b)
dans lequel un milieu de culture cellulaire, qui est, dans ce cas, un milieu d'alimentation, est ajouté au bioréacteur, de manière continue sur l'ensemble de la durée ou une ou plusieurs fois au cours de la durée d'incubation des cellules de l'étape c).

13. Procédé destiné à la culture de cellules selon l'une ou plusieurs parmi les revendications 7 à 11, **caractérisé en ce que** la quantité d'agent antimousse dans le milieu liquide est réduite par rapport à un milieu de culture cellulaire qui est par ailleurs identique mais qui comprend du poloxamère 188 au lieu d'un poloxamère ayant un bloc d'oxyde de polypropylène <700 g/mol et un pourcentage d'oxyde d'éthylène >45%.

14. Méthode de réduction de la formation de mousse dans des cultures cellulaires sous agitation et/ou barbotage, dans laquelle les cellules sont cultivées dans un milieu liquide comprenant un poloxamère ayant un bloc d'oxyde de polypropylène ≤ 700 g/mol et un pourcentage d'oxyde d'éthylène >45% selon une quantité comprise entre 0,1 et 10 g/L calculée pour le milieu liquide et la formation de mousse est réduite par rapport à des cellules cultivées dans les mêmes conditions dans un milieu de culture cellulaire qui comprend du poloxamère 188 au lieu d'un poloxamère ayant un bloc d'oxyde de polypropylène ≤700 g/mol et un pourcentage d'oxyde d'éthylène >45%.
